# EUROPEAN PATENT APPLICATION

(11) **EP 2 003 116 A2**
(43) Date of publication of application: **17.12.2008**
(21) Application number: 07741451.4
(22) Date of filing: 05.04.2007
(51) Int. Cl.: C07C 315/02, C07C 317/04, C07C 317/14, C07C 317/22, C07D 213/70, C07D 213/71, C07D 213/84, C07D 277/76, C07D 333/48, C07B 33/00

(54) **PROCESS FOR THE PRODUCTION OF ORGANIC OXIDES**

(30) Priority: 06.04.2006 JP 2006105160
(71) Applicant: Takeda Pharmaceutical Company Limited, Osaka-shi, Osaka 541-0045 (JP)
(72) Inventor: IKEMOTO, Tomomi, Osaka-shi Osaka 532-8686 (JP); FUKUDA, Naohiro, Osaka-shi Osaka 532-8686 (JP)
(74) Representative: Jones, Nicholas Andrew
(86) International application number: PCT/JP2007/058015
(87) International publication number: WO 2007/117027

(57) **Abstract**

The present invention provides a method for producing an organic oxide, wherein a substrate is oxidized using hypohalous acid, hypohalous acid salt, chlorine, bromine or iodine in the presence of water and a catalytic amount of a compound represented by the following formula (I): R¹-X¹-NY-R², wherein: X¹ represents -CO- or -SO₂-; Y represents a hydrogen atom, a potassium atom, a sodium atom, a chlorine atom, a bromine atom or an iodine atom; R¹ represents a substituted or unsubstituted hydrocarbon group, -NYR³ group or -OR³ group (in the formulae, R³ represents a substituted or unsubstituted hydrocarbon group, and Y represents the same meaning as defined above); and R² represents a hydrogen atom or -CO-R⁴ group (in the formula, R⁴ represents a substituted or unsubstituted hydrocarbon group, -NYR⁵ group or -OR⁵ group (in the formulae, R⁵ represents a substituted or unsubstituted hydrocarbon group, and Y represents the same meaning as defined above)); or R¹ and R⁴ may bind to each other to form a further substituted or unsubstituted nitrogen-containing heterocyclic ring.

## Description

### TECHNICAL FIELD

The present invention relates to a process for the production of organic oxides. More specifically, the present invention relates to a method for oxidizing an organic compound utilizing a catalyst.

### BACKGROUND ART

Organic oxides such as sulfoxide, sulfone, aldehyde, ketone and organic acids are used as various chemical products and synthetic intermediates thereof. As a method for producing these organic oxides, oxidation reaction is one of important reactions in the organic chemical industry. Among oxidation reactions, there are a wider variety of reactions using halogen derivatives. In particular, hypochlorite and N-halocarboxylic acid imide (e.g., N-chlorosuccinimide (NCS), N-bromosuccinimide (NBS), trichloroisocyanuric acid (TCCA) and tribromoisocyanuric acid (TBCA)) are frequently used as oxidants. For example, as methods for producing sulfone, the following reactions have been reported: oxidation reaction using sodium hypochlorite in which sulfide is used as a substrate (J. Am. Chem. Soc., 50, 1226 (1928)); oxidation reaction using sodium hypochlorite in the presence of a phase transfer catalyst (J. Am. Chem. Soc., 111, 3958 (1989)); oxidation reaction using sodium hypochlorite with pH adjusted (J. Chem. Soc. Chem. Comm., 1012 (1976)); and oxidation reaction using N-chlorosuccinimide (NCS) or N-bromosuccinimide (NBS) (J. Org. Chem., 33, 3996 (1968)).

However, from the viewpoint of industrial aspect, the number of sufficiently satisfying oxidation methods is small. For example, in the case of the above-described oxidation method using sodium hypochlorite, reaction velocity is very slow and a yield of sulfone is low, resulting in lower productivity. In the case of the improved methods using sodium hypochlorite in the presence of a phase transfer catalyst or with pH adjusted, yields are expected to be improved. However, since it is required to add a phase transfer catalyst or to adjust pH, operation is complicated. Moreover, since many oxidation reactions using hypochlorite are performed on the acidic side, there is a risk that hypochlorite may be decomposed during such reactions to generate chlorine gas. In the case of the oxidation reaction using N-halocarboxylic acid imide, it is superior to the case using sodium hypochlorite in terms of reaction time and oxide yield. However, there is a problem that carboxylic acid imide, which is a reaction by-product that is difficult to be removed, is generated and it makes isolation/purification of oxide difficult. Moreover, since these reactions are exothermic reactions, NCS, NBS or the like which is difficult to be dissolved in an organic solvent must be added slowly in the solid state, resulting in low operability of reaction. Furthermore, N-halocarboxylic acid imide is economically inefficient since it is expensive as a reagent.

### DISCLOSURE OF THE INVENTION

The purpose of the present invention is to provide an oxidation method which is superior to conventional oxidation methods in terms of reaction time, operability and safety of reaction, yield of reaction product, easiness of isolation/purification, economic efficiency, etc.

The present inventors diligently made researches to solve the above-described problems, and found the following matters: when oxidation reaction is performed using hypochlorite in the presence of a carboxylic acid imide catalyst, the reaction can be performed at lower temperatures in the alkaline state with good yield for shorter time compared to the conventional oxidation reactions using hypochlorite, and therefore it is excellent in operability and safety; when compared to the conventional oxidation reactions using N-halocarboxylic acid imide, since by-products are mineral salt and a catalytic amount of carboxylic acid imide, it is easier to perform isolation/purification, and high-quality oxides can be obtained; and in addition, the reaction is economically efficient since carboxylic acid imide is inexpensive compared to N-halocarboxylic acid imide. Moreover, the present inventors thought that when using N-halocarboxylic acid imide as a catalyst together with hypochlorite, the use amount of N-halocarboxylic acid imide can be significantly reduced compared to the conventional oxidation reactions only using N-halocarboxylic acid imide. Based on the above-described findings, the present inventors continued to make researches and completed the present invention.

Specifically, the present invention is as follows:
[1] A method for producing an organic oxide, wherein a substrate is oxidized using hypohalous acid, hypohalous acid salt, chlorine, bromine or iodine in the presence of water and a catalytic amount of a compound represented by the following formula (I):

   R¹-X¹-NY-R²,

   wherein:
   X¹ represents -CO- or -SO₂-; Y represents a hydrogen atom, a potassium atom, a sodium atom, a chlorine atom, a bromine atom or an iodine atom; R¹ represents a substituted or unsubstituted hydrocarbon group, -NYR³ group or -OR³ group (in the formulae, R³ represents a substituted or unsubstituted hydrocarbon group, and Y represents the same meaning as defined above); and R² represents a hydrogen atom or -CO-R⁴ group (in the formula, R⁴ represents a substituted or unsubstituted hydrocarbon group, -NYR⁵ group or -OR⁵ group (in the formulae, R⁵ represents a substituted or unsubstituted hydrocarbon group, and Y represents the same meaning as defined above)); or R¹ and R⁴ may bind to each other to form a further substituted or unsubstituted nitrogen-containing heterocyclic ring.
[2] The method according to item [1], wherein in the compound represented by formula (I), X¹ is -CO- and R² is -CO-R⁴ group (in the formula, R⁴ represents the same meaning as defined in item [1]).
[3] The method according to item [1], wherein the compound represented by formula (I) is a compound represented by the following formula (Ia): 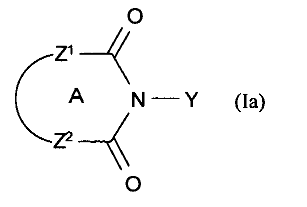 wherein: Ring A represents a further substituted or unsubstituted nitrogen-containing heterocyclic ring (in Ring A, Z¹ and Z² each independently represent a carbon atom, -NY- or -O-); and Y represents the same meaning as defined in item [1].
[4] The method according to item [3], wherein the compound represented by formula (Ia) is a compound represented by one of the following formulae: 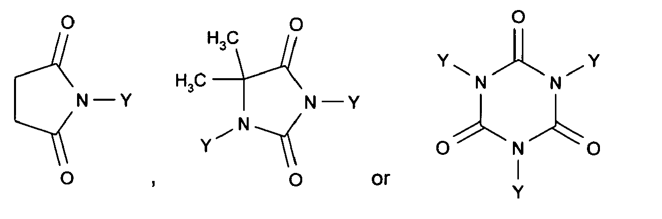 wherein Y represents the same meaning as defined in item [3].
[5] The method according to item [1], using a hypohalous acid salt selected from the group consisting of potassium hypochlorite, calcium hypochlorite, lithium hypochlorite, sodium hypobromite, calcium hypobromite, lithium hypobromite and sodium hypoiodite.
[6] The method according to item [1], wherein the substrate is sulfide or sulfoxide.

According to the present invention, an oxidation method, which is superior to conventional oxidation methods in terms of reaction time, yield, easiness of isolation/purification, operability and safety of reaction, economic efficiency, etc., is provided.

### BEST MODE FOR CARRYING OUT THE INVENTION

Hereinafter, the terms used herein will be explained.

As used herein, "organic oxides" mean sulfoxide, sulfone, aldehyde, ketone, organic acids, etc., unless otherwise specified.

In the present specification, examples of "hydrocarbon group" of "substituted or unsubstituted hydrocarbon group" include a C₁₋₁₀ alkyl group, a C₂₋₁₀ alkenyl group, a C₂₋₁₀ alkynyl group, a C₃₋₁₀ cycloalkyl group, a C₄₋₁₆ cycloalkylalkyl group, a C₃₋₁₀ cycloalkenyl group, a C₄₋₁₀ cycloalkadienyl group and a C₆₋₁₄ aryl group, unless otherwise specified.

Examples of C₁₋₁₀ alkyl groups include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl and decyl.

Examples of C₂₋₁₀ alkenyl groups include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl and 1-octenyl.

Examples of C₂₋₁₀ alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl and 1-octynyl.

Examples of C₃₋₁₀ cycloalkyl groups include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and adamantyl.

Examples of C₄₋₁₆ cycloalkylalkyl groups include cyclopropylmethyl, cyclopentylmethyl, cyclohexylmethyl and bicyclo[2.2.1]heptylmethyl.

Examples of C₃₋₁₀ cycloalkenyl groups include 2-cyclopentene-1-yl, 3-cyclopentene-1-yl, 2-cyclohexene-1-yl and 3-cyclohexene-1-yl.

Examples of C₄₋₁₀ cycloalkadienyl groups include 2,4-cyclopentadiene-1-yl, 2,4-cyclohexadiene-1-yl and 2,5-cyclohexadiene-1-yl.

Examples of C₆₋₁₄ aryl groups include phenyl, naphthyl, anthryl, phenanthryl, acenaphthylenyl and biphenylyl.

Examples of substituents which the "hydrocarbon group" of the "substituted or unsubstituted hydrocarbon group" may have include:
(1) a halogen atom (e.g., fluorine, chlorine, bromine and iodine);
(2) nitro;
(3) cyano;
(4) C₁₋₆ alkyl which may have a halogen atom (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl);
(5) C₂₋₆ alkenyl which may have a halogen atom (e.g., vinyl, allyl, isopropenyl, butenyl, isobutenyl, sec-butenyl, 3,3,3-trifluoro-1-propenyl and 4,4,4-trifluoro-1-butenyl);
(6) C₂₋₆ alkynyl which may have a halogen atom (e.g., ethynyl, propargyl, butynyl, 1-hexynyl, 3,3,3-trifluoro-1-propynyl and 4,4,4-trifluoro-1-butynyl);
(7) C₃₋₆ cycloalkyl (e.g., cyclopropyl, cyclobutyl, cyclopentyl and cyclohexyl);
(8) C₆₋₁₄ aryl (e.g., phenyl, 1-naphthyl, 2-naphthyl, biphenylyl and 2-anthryl);
(9) C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl and 5-phenylpentyl);
(10) hydroxy;
(11) C₁₋₆ alkoxy (e.g., methoxy, ethoxy, propoxy, isopropoxy, butoxy, isobutoxy, sec-butoxy, pentyloxy and hexyloxy);
(12) C₆₋₁₄ aryloxy (e.g., phenyloxy and naphthyloxy);
(13) mercapto;
(14) C₁₋₆ alkylthio (e.g., methylthio, ethylthio, propylthio, isopropylthio, butylthio, pentylthio and hexylthio);
(15) C₆₋₁₄ arylthio (e.g., phenylthio and naphthylthio);
(16) amino;
(17) mono-C₁₋₆ alkylamino (methylamino and ethylamino);
(18) mono-C₆₋₁₄ arylamino (e.g., phenylamino, 1-naphthylamino and 2-naphthylamino);
(19) di-C₁₋₆ alkylamino (e.g., dimethylamino and diethylamino);
(20) di-C₆₋₁₄ arylamino (e.g., diphenylamino);
(21) formyl;
(22) C₁₋₆ alkyl-carbonyl (e.g., acetyl and propionyl);
(23) C₆₋₁₄ aryl-carbonyl (e.g., benzoyl, 1-naphthoyl and 2-naphthoyl);
(24) carboxy;
(25) C₁₋₆ alkoxy-carbonyl (e.g., methoxycarbonyl, ethoxycarbonyl, propoxycarbonyl and tert-butoxycarbonyl);
(26) C₆₋₁₄ aryloxy-carbonyl (e.g., phenoxycarbonyl);
(27) carbamoyl;
(28) thiocarbamoyl;
(29) mono-C₁₋₆ alkyl-carbamoyl (e.g., methylcarbamoyl and ethylcarbamoyl);
(30) di-C₁₋₆ alkyl-carbamoyl (e.g., dimethylcarbamoyl, diethylcarbamoyl and ethylmethylcarbamoyl);
(31) C₆₋₁₄ aryl-carbamoyl (e.g., phenylcarbamoyl, 1-naphthylcarbamoyl and 2-naphthylcarbamoyl);
(32) C₁₋₆ alkylsulfonyl (e.g., methylsulfonyl and ethylsulfonyl);
(33) C₆₋₁₄ arylsulfonyl (e.g., phenylsulfonyl, 1-naphthylsulfonyl and 2-naphthylsulfonyl);
(34) C₁₋₆ alkylsulfinyl (e.g., methylsulfinyl and ethylsulfinyl);
(35) C₆₋₁₄ arylsulfinyl (e.g., phenylsulfinyl, 1-naphthylsulfinyl and 2-naphthylsulfinyl);
(36) formylamino;
(37) C₁₋₆ alkyl-carbonylamino (e.g., acetylamino);
(38) C₆₋₁₄ aryl-carbonylamino (e.g., benzoylamino and naphthoylamino);
(39) C₁₋₆ alkoxy-carbonylamino (e.g., methoxycarbonylamino, ethoxycarbonylamino, propoxycarbonylamino and butoxycarbonylamino);
(40) C₁₋₆ alkylsulfonylamino (e.g., methylsulfonylamino and ethylsulfonylamino);
(41) C₆₋₁₄ arylsulfonylamino (e.g., phenylsulfonylamino, 2-naphthylsulfonylamino and 1-naphthylsulfonylamino);
(42) C₁₋₆ alkyl-carbonyloxy (e.g., acetoxy and propionyloxy);
(43) C₆₋₁₄ aryl-carbonyloxy (e.g., benzoyloxy and naphthylcarbonyloxy);
(44) C₁₋₆ alkoxy-carbonyloxy (e.g., methoxycarbonyloxy, ethoxycarbonyloxy, propoxycarbonyloxy and butoxycarbonyloxy);
(45) mono-C₁₋₆ alkyl-carbamoyloxy (e.g., methylcarbamoyloxy and ethylcarbamoyloxy);
(46) di-C₁₋₆ alkyl-carbamoyloxy (e.g., dimethylcarbamoyloxy and diethylcarbamoyloxy);
(47) C₆₋₁₄ aryl-carbamoyloxy (e.g., phenylcarbamoyloxy and naphthylcarbamoyloxy);
(48) 5- to 7-membered saturated cyclic amino which may comprise one or two types of 1 to 4 hetero atoms selected from a nitrogen atom, a sulfur atom and an oxygen atom other than one nitrogen atom and carbon atoms (e.g., pyrrolidin-1-yl, piperidino, piperazin-1-yl, morpholino, thiomorpholino and hexahydroazepin-1-yl);
(49) 5- to 10-membered aromatic heterocyclic group which comprises one or two types of 1 to 4 hetero atoms selected from a nitrogen atom, a sulfur atom and an oxygen atom other than carbon atoms (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl and 3-benzo[b]furanyl); and
(50) oxo.

When the "hydrocarbon group" of the "substituted or unsubstituted hydrocarbon group" has the above-described "substituents," the group may have 1 to 5 such substituents, and preferably 1 to 3 such substituents at replaceable positions. When the number of substituents is 2 or more, such substituents may be the same or different.

In the present specification, examples of "heterocyclic group" of "substituted or unsubstituted heterocyclic group" include an aromatic heterocyclic group and a non-aromatic heterocyclic group, unless otherwise specified. In this regard, examples of aromatic heterocyclic groups include a 5- to 7-membered monocyclic aromatic heterocyclic group which comprises 1 to 4 hetero atoms selected from an oxygen atom, a sulfur atom and a nitrogen atom as ring-constituting atoms other than carbon atoms, and a condensed aromatic heterocyclic group. Examples of condensed aromatic heterocyclic groups include groups in which one or two 5- or 6-membered rings comprising 1 or 2 nitrogen atoms, 5-membered rings or benzene rings comprising one sulfur atom or the like are condensed with the 5- to 7-membered monocyclic aromatic heterocyclic group.

Suitable examples of aromatic heterocyclic groups include: monocyclic aromatic heterocyclic groups such as furyl (e.g., 2-furyl and 3-furyl), thienyl (e.g., 2-thienyl and 3-thienyl), pyridyl (e.g., 2-pyridyl, 3-pyridyl and 4-pyridyl), pyrimidinyl (e.g., 2-pyrimidinyl, 4-pyrimidinyl, 5-pyrimidinyl and 6-pyrimidinyl), pyridazinyl (e.g., 3-pyridazinyl and 4-pyridazinyl), pyrazinyl (e.g., 2-pyrazinyl), pyrrolyl (e.g., 1-pyrrolyl, 2-pyrrolyl and 3-pyrrolyl), imidazolyl (e.g., 1-imidazolyl, 2-imidazolyl, 4-imidazolyl and 5-imidazolyl), pyrazolyl (e.g., 1-pyrazolyl, 3-pyrazolyl and 4-pyrazolyl), thiazolyl (e.g., 2-thiazolyl, 4-thiazolyl and 5-thiazolyl), isothiazolyl (e.g., 4-isothiazolyl), oxazolyl (e.g., 2-oxazolyl, 4-oxazolyl and 5-oxazolyl), isoxazolyl, oxadiazolyl (e.g., 1,2,4-oxadiazol-5-yl and 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl and 1,2,3-triazol-4-yl), tetrazolyl (e.g., tetrazol-1-yl and tetrazol-5-yl), and triazinyl (e.g., 1,2,4-triazol-1-yl and 1,2,4-triazol-3-yl); and condensed aromatic heterocyclic groups such as quinolyl (e.g., 2-quinolyl, 3-quinolyl, 4-quinolyl and 6-quinolyl), isoquinolyl (e.g., 3-isoquinolyl), quinazolyl (e.g., 2-quinazolyl and 4-quinazolyl), quinoxalyl (e.g., 2-quinoxalyl and 6-quinoxalyl), benzofuryl (e.g., 2-benzofuryl and 3-benzofuryl), benzothienyl (e.g., 2-benzothienyl and 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl and benzimidazol-5-yl), benzotriazolyl (e.g., 1H-1,2,3-benzotriazol-5-yl), indolyl (e.g., indol-1-yl, indol-2-yl, indol-3-yl and indol-5-yl), indazolyl (e.g., 1H-indazol-3-yl), pyrrolopyrazinyl (e.g., 1H-pyrrolo[2,3-b]pyrazin-2-yl and 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl and 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), and pyrazolotriazinyl (e.g., pyrazolo[5,1-c][1,2,4]triazin-3-yl).

Examples of non-aromatic heterocyclic groups include a 5- to 7-membered monocyclic non-aromatic heterocyclic group which comprises 1 to 4 hetero atoms selected from an oxygen atom, a sulfur atom and a nitrogen atom as ring-constituting atoms other than carbon atoms, and a condensed non-aromatic heterocyclic group. Examples of condensed non-aromatic heterocyclic groups include groups in which one or two 5- or 6-membered rings comprising 1 or 2 nitrogen atoms, 5-membered rings or benzene rings comprising one sulfur atom or the like are condensed with the 5- to 7-membered monocyclic non-aromatic heterocyclic group.

Suitable examples of non-aromatic heterocyclic groups include pyrrolidinyl (e.g., 1-pyrrolidinyl), piperidinyl (e.g., piperidino), morpholinyl (e.g., morpholino), thiomorpholinyl (e.g., thiomorpholino), piperazinyl (e.g., 1-piperazinyl), hexamethyleneiminyl (e.g., hexamethyleneimin-1-yl), oxazolidinyl (e.g., oxazolidin-3-yl), thiazolidinyl (e.g., thiazolidin-3-yl), imidazolidinyl (e.g., imidazolidin-3-yl), dihydroisoindolyl (e.g., 1,3-dihydro-2H-isoindol-2-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, tetrahydropyranyl (e.g., 4-tetrahydropyranyl), dihydrobenzofuranyl (e.g., 2,3-dihydro-1-benzofuran-5-yl), dihydrobenzodioxin (e.g., 2,3-dihydro-1,4-benzodioxin), dihydrobenzodioxepin (e.g., 3,4-dihydro-2H-1,5-benzodioxepin), 4,5,6,7-tetrahydro-1-benzofuranyl (e.g., 4,5,6,7-tetrahydro-1-benzofuran-3-yl), indanyl (e.g., indan-5-yl), chromenyl (e.g., 4H-chromen-2-yl and 2H-chromen-3-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), pyrazolidinyl (e.g., pyrazolidin-1-yl), and tetrahydroquinolinyl (e.g., 1,2,3,4-tetrahydroquinolin-4-yl).

The aforementioned "heterocyclic group" of "substituted or unsubstituted heterocyclic group" may have 1 to 3 substituents at replaceable positions. Examples of the substituents include those exemplified as the substituents which the "hydrocarbon group" of the "substituted or unsubstituted hydrocarbon group" may have.

In the present specification, examples of "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl, unless otherwise specified.

As a catalyst in the oxidation method of the present invention, a compound represented by formula (I): R¹-X¹-NY-R² (hereinafter also referred to as Compound (I)) is used, wherein in the formula: X¹ represents -CO- or -SO₂-; Y represents a hydrogen atom, a potassium atom, a sodium atom, a chlorine atom, a bromine atom or an iodine atom; R¹ represents a substituted or unsubstituted hydrocarbon group, -NYR³ group or -OR³ group (in the formulae, R³ represents a substituted or unsubstituted hydrocarbon group, and Y represents the same meaning as defined above); and R² represents a hydrogen atom or -CO-R⁴ group (in the formula, R⁴ represents a substituted or unsubstituted hydrocarbon group, -NYR⁵ group or -OR⁵ group (in the formulae, R⁵ represents a substituted or unsubstituted hydrocarbon group, and Y represents the same meaning as defined above)); or R¹ and R⁴ may bind to each other to form a further substituted or unsubstituted nitrogen-containing heterocyclic ring.

In the above-described formula (I), as the "substituted or unsubstituted hydrocarbon groups" represented by R¹, R³, R⁴ and R⁵, those defined above are used.

When R¹ is a "substituted or unsubstituted hydrocarbon group," R¹ is preferably a C₁₋₁₀ alkyl group or a C₆₋₁₄ aryl group. R¹ is particularly preferably a C₁₋₆ alkyl group or a phenyl group.

When R¹ is "-NYR³ group" or "-OR³ group," the "substituted or unsubstituted hydrocarbon group" represented by R³ is preferably a C₁₋₁₀ alkyl group, and particularly preferably a C₁₋₆ alkyl group.

When R² is "-CO-R⁴ group" and R⁴ is a "substituted or unsubstituted hydrocarbon group," R⁴ is preferably a C₁₋₁₀ alkyl group or a C₆₋₁₄ aryl group, and particularly preferably a C₁₋₆ alkyl group or a phenyl group.

When R² is "-CO-R⁴ group" and R⁴ is "-NYR⁵ group" or "-OR⁵ group," the "substituted or unsubstituted hydrocarbon group" represented by R⁵ is preferably a C₁₋₁₀ alkyl group, and particularly preferably a C₁₋₆ alkyl group.

In the above-described formula (I), Y is preferably a hydrogen atom, a chlorine atom, a bromine atom or an iodine atom.

In the above-described formula (I), examples of the "nitrogen-containing heterocyclic ring" of the "further substituted or unsubstituted nitrogen-containing heterocyclic ring," which R¹ and R⁴ bind to each other to form, include a 5- to 7-membered monocyclic non-aromatic heterocyclic ring which comprises at least one nitrogen atom and further comprises 1 to 4 hetero atoms selected from an oxygen atom, a sulfur atom and a nitrogen atom as ring-constituting atoms other than carbon atoms, and a condensed non-aromatic heterocyclic group. Examples of condensed non-aromatic heterocyclic rings include rings in which one or two 5- or 6-membered rings comprising 1 or 2 nitrogen atoms, 5-membered rings comprising one sulfur atom, cyclohexanes, cyclohexenes or benzene rings or the like are condensed with the 5- to 7-membered monocyclic non-aromatic heterocyclic ring.

Preferred specific examples of structures of the "nitrogen-containing heterocyclic ring" of the "further substituted or unsubstituted nitrogen-containing heterocyclic ring," which R¹ and R⁴ bind to each other to form, include: 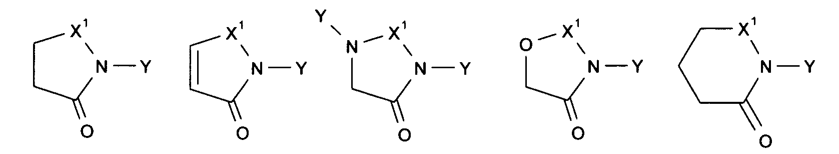 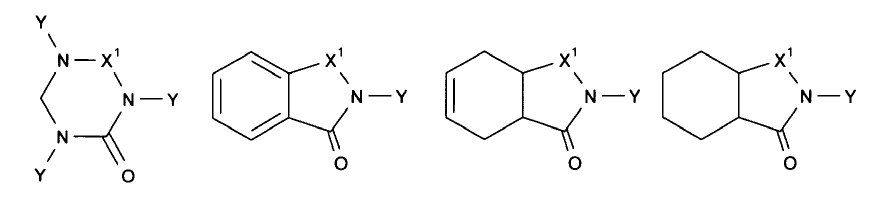 wherein each symbol represents the same meaning as defined above.

The above-described "nitrogen-containing heterocyclic ring" of the "further substituted or unsubstituted nitrogen-containing heterocyclic ring" may further have 1 to 3 (preferably 1 or 2) substituents at replaceable positions other than a nitrogen atom. As the substituents, those similar to the "substituents" of the "substituted or unsubstituted heterocyclic ring" are used. Among them, a C₁₋₆ alkyl group, an oxo group, a halogen atom or an amino group is preferred. When there are 2 or more substituents, they may be the same or different.

In one embodiment of the present invention, Compound (I), wherein in formula (I), X¹ is -CO- and R² is a hydrogen atom, is used. Specific examples of Compound (I) include acetamide, benzamide, and compounds in which a hydrogen atom of these amino groups is substituted with a potassium atom, a sodium atom, a chlorine atom, a bromine atom or an iodine atom (e.g., N-chloroacetamide and N-bromoacetamide).

In another embodiment of the present invention, Compound (I), wherein in formula (I), X¹ is -CO- and R² is -CO-R⁴ group (the definition of R⁴ is as described above), is used. Specific examples of Compound (I) include diacetamide, acetylurethane, succinimide, 5,5-dimethylhydantoin, cyanuric acid, maleimide, hydantoin, glutarimide, 2,4-dioxohexahydro-1,3,5-triazine, phthalimide, 1,2,3,6-tetrahydrophthalimide, hexahydrophthalimide, 5,5-dimethyloxazolidine-2,4-dione, and compounds in which a hydrogen atom of these imino groups is substituted with a potassium atom, a sodium atom, a chlorine atom, a bromine atom or an iodine atom (e.g., N-chlorosuccinimide (NCS), N-bromosuccinimide (NBS), trichloroisocyanuric acid (TCCA), tribromoisocyanuric acid (TBCA), sodium dichloroisocyanurate, potassium dichloroisocyanurate, sodium phthalimide, and potassium phthalimide).

In still another embodiment, Compound (I), wherein in formula (I), X¹ is -SO₂- and R² is a hydrogen atom, is used. Specific examples of these compounds include methanesulfonamide, benzenesulfonamide, p-toluenesulfonamide, 10-camphorsulfonamide, and compounds in which a hydrogen atom of these amino groups is substituted with a potassium atom, a sodium atom, a chlorine atom, a bromine atom or an iodine atom (e.g., N-chlorobenzenesulfonamide, N-chloro-p-toluenesulfonamide, and N-bromo-p-toluenesulfonamide).

In still another embodiment, Compound (I), wherein in formula (I), X¹ is -SO₂- and R² is -CO-R⁴ group (the definition of R⁴ is as described above), is used. Examples of Compound (I) include N-(tert-butoxycarbonyl)-p-toluenesulfonamide, saccharin, 5-chlorosaccharin, 5-aminosaccharin, and compounds in which a hydrogen atom of these imino groups is substituted with a potassium atom, a sodium atom, a chlorine atom, a bromine atom or an iodine atom (e.g., N-chlorosaccharin and N-bromosaccharin).

In a preferred embodiment, Compound (I), wherein in formula (I), X¹ is -CO- and R² is -CO-R⁴ group (the definition of R⁴ is as described above), is used.

In a more preferred embodiment, a compound represented by the following formula (Ia) (also referred to as "Compound (Ia)" herein): 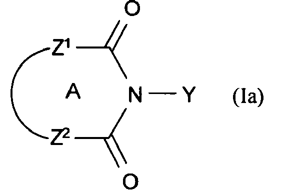 wherein Ring A represents a further substituted or unsubstituted nitrogen-containing heterocyclic ring (in Ring A, Z¹ and Z² each independently represent a carbon atom, -NY- or -O-); and Y represents the same meaning as described above), is used as Compound (I), wherein: X¹ is -CO-; R² is -CO-R⁴ group; and R¹ and R⁴ bind to each other to form a further substituted or unsubstituted nitrogen-containing heterocyclic ring.

In the above-described formula (Ia), as the "nitrogen-containing heterocyclic ring" of the "further substituted or unsubstituted nitrogen-containing heterocyclic ring" represented by Ring A, rings, which are similar to the "nitrogen-containing heterocyclic ring" of the "further substituted or unsubstituted nitrogen-containing heterocyclic ring" formed by the binding of R¹ and R⁴, except that X¹ in formula (I) is -CO-, are used.

Specifically, preferred examples of structures of the "nitrogen-containing heterocyclic ring" of the "further substituted or unsubstituted nitrogen-containing heterocyclic ring" represented by Ring A include: 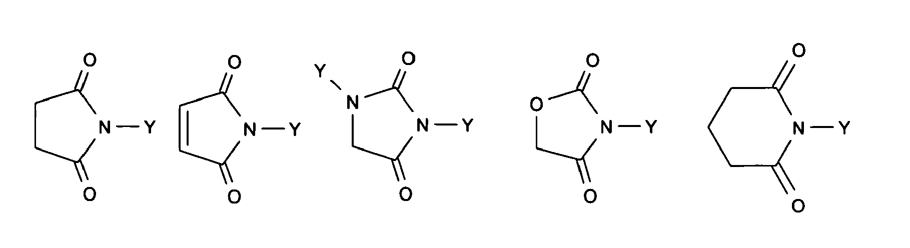 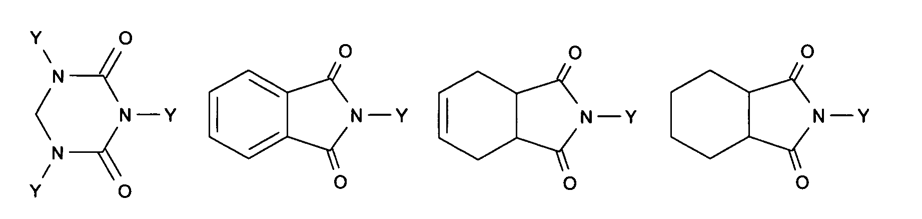 wherein Y represents the same meaning as defined above.

The above-described "nitrogen-containing heterocyclic ring" of the "further substituted or unsubstituted nitrogen-containing heterocyclic ring" may further have 1 to 3 (preferably 1 or 2) substituents at replaceable positions other than a nitrogen atom. As such substituents, those similar to the "substituents" of the "nitrogen-containing heterocyclic ring" of the "further substituted or unsubstituted nitrogen-containing heterocyclic ring" formed by the binding of R¹ and R⁴ are used. Among them, a C₁₋₆ alkyl group or an oxo group is preferred. When the number of substituents is 2 or more, such substituents may be the same or different.

As Compound (Ia), compounds represented by the following formulae: 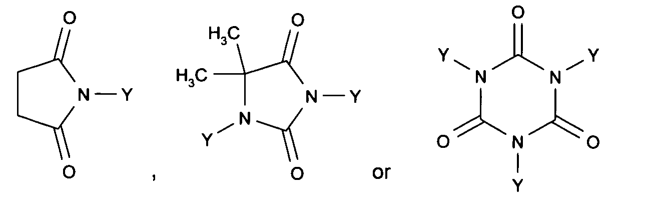 wherein each symbol represents the same meaning as defined above, are preferred.

In the above-described formula (Ia), Y is preferably a hydrogen atom, a chlorine atom, a bromine atom or an iodine atom.

Specific examples of Compound (Ia) include succinimide, 5,5-dimethylhydantoin, cyanuric acid, maleimide, hydantoin, glutarimide, 2,4-dioxohexahydro-1,3,5-triazine, phthalimide, 1,2,3,6-tetrahydrophthalimide, hexahydrophthalimide, 5,5-dimethyloxazolidine-2,4-dione, and compounds in which a hydrogen atom of these imino groups is substituted with a potassium atom, a sodium atom, a chlorine atom, a bromine atom or an iodine atom (e.g., N-chlorosuccinimide (NCS), N-bromosuccinimide (NBS), trichloroisocyanuric acid (TCCA), tribromoisocyanuric acid (TBCA), sodium dichloroisocyanurate, potassium dichloroisocyanurate, sodium phthalimide, and potassium phthalimide). Among them, succinimide, 5,5-dimethylhydantoin, cyanuric acid, N-chlorosuccinimide (NCS), N-bromosuccinimide (NBS), trichloroisocyanuric acid (TCCA), tribromoisocyanuric acid (TBCA), sodium dichloroisocyanurate, potassium dichloroisocyanurate, sodium phthalimide or potassium phthalimide is preferred.

Compounds represented by formula (I) or (Ia) can be produced according to publicly-known methods of organic chemical synthesis. Alternatively, various compounds represented by formula (I) or (Ia) (e.g., the compounds listed as specific examples) are commercially available.

In the oxidation method of the present invention, hypohalous acid, hypohalous acid salt, chlorine, bromine or iodine acts as a cooxidant.

Examples of hypohalous acids include hypochlorous acid, hypobromous acid, and hypoiodous acid.

Examples of hypohalous acid salts include potassium hypochlorite, calcium hypochlorite, lithium hypochlorite, sodium hypobromite, calcium hypobromite, lithium hypobromite, and sodium hypoiodite. Such hypohalous acid salts are commercially available in the form of solid or aqueous solution thereof.

As the cooxidant to be used in the oxidation method of the present invention, hypohalous acid salt is preferred.

The oxidation method of the present invention can be applied to various oxidation reactions performed using halogens.

In one aspect, the present invention can be used to produce sulfoxide or sulfone utilizing sulfide as a substrate or to produce sulfone utilizing sulfoxide as a substrate. A typical method for producing sulfoxide or sulfone using the oxidation method of the present invention is shown in the below scheme 1.

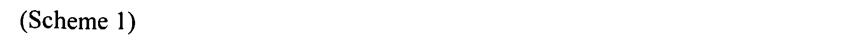

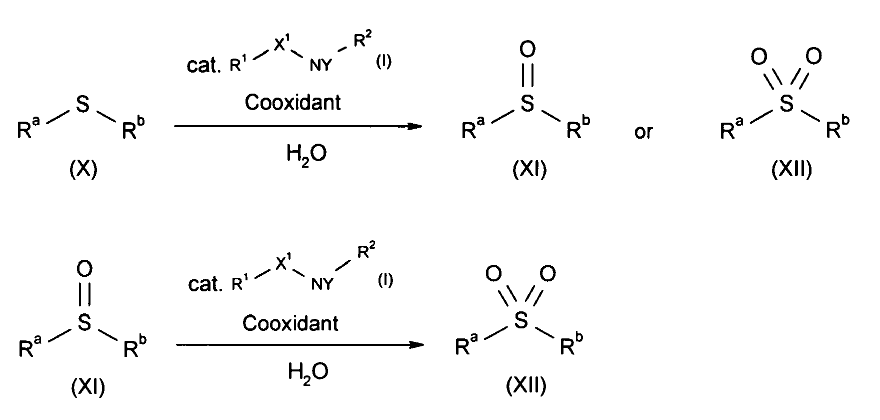

In the above reaction formula, R^{a} and R^{b} identically or differently represent a group that binds via a carbon atom. Alternatively, R^{a} and R^{b} may bind to each other to form a substituted or unsubstituted sulfur-containing heterocyclic ring. The "cooxidant" represents hypohalous acid, hypohalous acid salt, chlorine, bromine or iodine. Each of the other symbols represents the same meaning as described above.

Examples of the "group that binds via a carbon atom" represented by R^{a} and R^{b} include a substituted or unsubstituted hydrocarbon group, and a substituted or unsubstituted heterocyclic group having a bond at a carbon atom.

Examples of the above-described "substituted or unsubstituted hydrocarbon group" include those defined above.

Examples of the above-described "substituted or unsubstituted heterocyclic group having a bond at a carbon atom" include a heterocyclic group having a bond at a carbon atom which constitutes a heterocyclic ring of the aforementioned "substituted or unsubstituted heterocyclic group."

Examples of the "sulfur-containing heterocyclic ring" of the "substituted or unsubstituted sulfur-containing heterocyclic ring" formed by the binding of R^{a} and R^{b} include a 4- to 8-membered heterocyclic ring which may contain 1 to 3 hetero atoms (e.g., nitrogen, oxygen and sulfur) other than a sulfur atom (e.g., tetrahydrothiophene and pentamethylenesulfide). The "sulfur-containing heterocyclic ring" may be condensed with a 5- or 6-membered ring (e.g., benzene, pyridine, pyrazine, pyrimidine, pyridazine and cyclohexane) to form a dicyclic condensed ring.

The above-described "sulfur-containing heterocyclic ring" may further have 1 to 3 (preferably 1 or 2) substituents at replaceable positions. As such substituents, those similar to the "substituents" of the "substituted or unsubstituted heterocyclic ring" can be used.

In the reaction shown in Scheme 1, Compound (I), water and a cooxidant are added to sulfide (X) as a substrate, and the mixture is reacted to obtain sulfoxide (XI) or sulfone (XII) as a substrate. Alternatively, Compound (I), water and a cooxidant are added to sulfoxide (XI), and the mixture is reacted to obtain sulfone (XII).

In another aspect, the present invention can be used to produce aldehyde or ketone or organic acid using alcohol as a substrate or to produce organic acid using aldehyde as a substrate. A typical method for producing aldehyde or ketone or organic acid using the oxidation method of the present invention is shown in the below scheme 2.

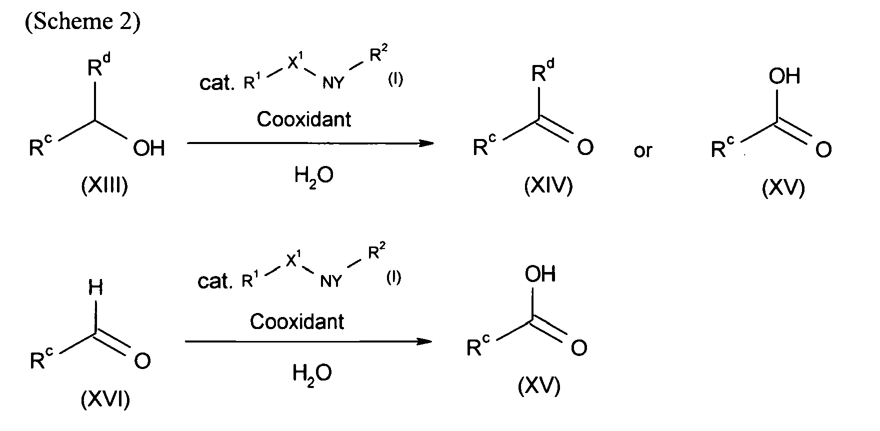

In the above-described reaction formula: R^{c} represents a group that binds via a carbon atom; R^{d} represents a hydrogen atom or a group that binds via a carbon atom; the "cooxidant" represents hypohalous acid, hypohalous acid salt, chlorine, bromine or iodine; and each of the other symbols represents the same meaning as described above.

Examples of the "group that binds via a carbon atom" represented by R^{c} and R^{d} include a substituted or unsubstituted hydrocarbon group, or a substituted or unsubstituted heterocyclic group having a bond at a carbon atom.

Examples of the above-described "substituted or unsubstituted hydrocarbon group" include those defined above.

Examples of the above-described "substituted or unsubstituted heterocyclic group having a bond at a carbon atom" include a heterocyclic group having a bond at a carbon atom which constitutes a heterocyclic ring of the aforementioned "substituted or unsubstituted heterocyclic group."

In the reaction shown in Scheme 2, Compound (I), water and a cooxidant are added to alcohol (XIII) as a substrate, and the mixture is reacted to obtain aldehyde or ketone (XIV) or organic acid (XV). Alternatively, Compound (I), water and a cooxidant are added to aldehyde (XVI) as a substrate, and the mixture is reacted to obtain organic acid (XV).

Further, the present invention can also be applied to oxidation reactions, which have already been reported regarding N-haloimide that is a catalyst in the oxidation reaction of the present invention, e.g., halogenation (Synthesis, 2006, 221-223), deprotection (J. Org. Chem., 1974, 39, 3504-3506; Synthesis, 1992, 439-442); and oxidation (Org. Lett., 2001, 3, 3041-3043; J. Org. Chem., 2003, 68, 4999-5001; Org. Process Res. & Dev., 2004, 8, 931-938).

The use amount of Compound (I), which is a catalyst in the oxidation method of the present invention, may be an amount by which catalyst action can be obtained (that is, a catalytic amount), and it depends on a substrate used, reaction condition, etc. In general, the use amount is less than 1 mol, e.g., 0.001 to 0.99 mol, preferably 0.001 to 0.5 mol, and more preferably 0.001 to 0.3 mol per 1 mol of substrate. Though the oxidation method of the present invention can be performed outside this range, reaction time increases when the use amount is less than 0.001 mol, and it is time-consuming to separate Compound (I) from a reaction product when the use amount is 1 mol or more.

The use amount of hypohalous acid, hypohalous acid salt, chlorine, bromine or iodine, which is a cooxidant in the oxidation method of the present invention, depends on the type of cooxidant, the type of organic oxide to be targeted, the type of Compound (I) to be used as a catalyst, reaction conditions, etc. In general, the use amount is 0.2 to 10.0 mol, preferably 0.5 to 5.0 mol, and more preferably 1.0 to 5.0 mol per 1 mol of substrate.

A solvent in the oxidation method of the present invention is not particularly limited as long as it does not affect reactions. Examples thereof include hydrocarbon-based solvents (e.g., hexane, heptane and toluene), ether-based solvents (e.g., dimethoxyethane, diethyl ether, diisopropyl ether, tetrahydrofuran and cyclopentylmethyl ether), ester-based solvents (e.g., ethyl acetate, methyl acetate and butyl acetate), acetonitrile, dimethylformamide, dimethylacetamide, methylisobutylketone, acetone, and methyl ethyl ketone. Among them, toluene, dimethoxyethane, tetrahydrofuran, ethyl acetate and acetonitrile are preferred. One of them can be used solely. Alternatively, two or more of them can be used by mixing them in a suitable ratio. Alternatively, the present oxidation method can be practiced without a solvent. The use amount of the solvent depends on the type of substrate, reaction conditions, etc. In general, the use amount is 1 to 100 mL, and preferably 5 to 20 mL per 1 g of substrate.

In the oxidation method of the present invention, water can be used by directly adding to a reaction solvent. Alternatively, an aqueous solution of hypochlorous acid or hypohalous acid salt can be used. Alternatively, water can be used as a reaction solvent. The use amount of water in the present oxidation method depends on the type of substrate, the type of solvent to be used, reaction conditions, etc. In general, the use amount is 0.5 to 1000 mL, and preferably 5 to 20 mL per 1 g of substrate.

Reaction pH of the oxidation method of the present invention is usually pH 7 to 14, and preferably pH 9 to 14.

Reaction temperature of the oxidation method of the present invention depends on the type of substrate to be used, the type of reagent, the type of solvent, etc. In general, the reaction temperature is -20 to 130°C, and preferably 0 to 50°C. Reaction time depends on the type of substrate to be used, the type of reagent, the type of solvent, etc. Usually, the reaction time is 0.5 to 10 hours, and preferably 0.5 to 5 hours. Further, the present oxidation method is usually practiced under ordinary pressure.

The organic oxide obtained according to the oxidation method of the present invention can be separated and purified using a publicly-known means, e.g., a separation method such as solvent extraction, concentration, filtration, crystallization and chromatography, and a method consisting of a combination thereof.

Hereinafter, the present invention will be specifically described by way of examples. However, the Examples described below are provided only for illustrative purposes, and do not limit the scope of the present invention.

### EXAMPLES

### (Example 1)

Diphenylsulfide (1.00 g, 5.37 mmol) and cyanuric acid (69 mg, 0.54 mmol) were mixed with toluene (20 mL). 10% aqueous solution of sodium hypochlorite (12.0 g, 16.11 mmol) was added to the mixture at room temperature, and the obtained mixture was stirred for 2 hours. After that, sodium sulfite (677 mg, 5.37 mmol) and water (10 mL) were added to the reaction mixture, and it was extracted with ethyl acetate (10 mL). An organic layer was concentrated under reduced pressure, and the obtained residue was subjected to purification by means of a silica gel column to obtain diphenylsulfone (1.10 g, yield: 94%).
¹H-NMR (300MHz, CDCl₃) δ 7.42-7.65 (6H, m), 7.90-7.99 (4H, m)

### (Example 2)

Diphenylsulfide (1.00 g, 5.37 mmol) and trichloroisocyanuric acid (125 mg, 0.54 mmol) were mixed with toluene (20 mL). 10% aqueous solution of sodium hypochlorite (12.0 g, 16.11 mmol) was added to the mixture at room temperature, and the obtained mixture was stirred for 3 hours. After that, sodium sulfite (2.03 g, 16.11 mmol) and water (10 mL) were added to the reaction mixture, and it was extracted with ethyl acetate (10 mL). An organic layer was concentrated under reduced pressure, and the obtained residue was subjected to purification by means of a silica gel column to obtain diphenylsulfone (1.10 g, yield: 94%).

### (Example 3)

Diphenylsulfide (1.00 g, 5.37 mmol) and acetamide (32 mg, 0.54 mmol) were mixed with dimethoxyethane (20mL). 10% aqueous solution of sodium hypochlorite (12.0 g, 16.11 mmol) was added to the mixture at room temperature, and the obtained mixture was stirred for 4 hours. After that, ethyl acetate (20 mL), water (20 mL) and sodium sulfite (2.03 g, 16.11 mmol) were added to the reaction mixture. The obtained mixture was stirred for 30 minutes and thereafter it was subjected to separation. An organic layer was subjected to quantitative determination using HPLC, and it was confirmed that diphenylsulfone (313 mg, yield: 27%) and diphenylsulfoxide (768 mg, yield: 71 %) were produced.

### (Example 4)

Diphenylsulfide (1.00 g, 5.37 mmol) and p-toluenesulfonamide (92 mg, 0.54 mmol) were mixed with dimethoxyethane (20 mL). 10% aqueous solution of sodium hypochlorite (12.0 g, 16.11 mmol) was added to the mixture at room temperature, and the obtained mixture was stirred for 4 hours. After that, ethyl acetate (20 mL), water (20 mL) and sodium sulfite (2.03 g, 16.11 mmol) were added to the reaction mixture. The obtained mixture was stirred for 30 minutes and thereafter it was subjected to separation. An organic layer was subjected to quantitative determination using HPLC, and it was confirmed that diphenylsulfone (910 mg, yield: 27%) and diphenylsulfoxide (190 mg, yield: 71 %) were produced.

### (Example 5)

Dibutylsulfide (1.00 g, 6.84 mmol) and cyanuric acid (88 mg, 0.68 mmol) were mixed with toluene (20 mL). 10% aqueous solution of sodium hypochlorite (11.7 g, 15.7 mmol) was added to the mixture at room temperature, and the obtained mixture was stirred for 1 hour. After that, sodium sulfite (431 mg, 3.42 mmol) and water (10 mL) were added to the reaction mixture, and it was extracted with ethyl acetate (10 mL). An aqueous layer was extracted with ethyl acetate (10 mL), and a combined organic layer was washed with saturated sodium chloride solution (10 mL). The organic layer was concentrated under reduced pressure, and the obtained residue was subjected to purification by means of a silica gel column to obtain dibutylsulfone (1.17 g, yield: 96%).
¹H-NMR (300MHz, CDCl₃) δ 0.97 (6H, t), 1.43-1.55 (4H, m), 1.76-1.88 (4H, m), 2.91-2.98 (4H, m)

### (Example 6)

(Methylthio)benzene (1.00 g, 8.05 mmol) and cyanuric acid (104 mg, 0.81 mmol) were mixed with toluene (20 mL). 10% aqueous solution of sodium hypochlorite (13.8 g, 18.5 mmol) was added to the mixture at room temperature, and the obtained mixture was stirred for 1 hour. After that, sodium sulfite (507 mg, 4.03 mmol) and water (10 mL) were added to the reaction mixture, and it was extracted with ethyl acetate (10 mL). An aqueous layer was extracted with ethyl acetate (10 mL), and a combined organic layer was washed with saturated saline (10 mL). The organic layer was concentrated under reduced pressure, and the obtained residue was subjected to purification by means of a silica gel column to obtain (methylsulfonyl)benzene (1.21 g, yield: 96%).
¹H-NMR (300MHz, CDCl₃) δ 3.06 (3H, s), 7.54-7.68 (3H, m), 7.93-7.98 (2H, m)

### (Example 7)

(Benzylthio)benzene (500 mg, 2.50 mmol) and cyanuric acid (32 mg, 0.25 mmol) were mixed with toluene (10 mL). 10% aqueous solution of sodium hypochlorite (5.57 g, 7.49 mmol) was added dropwise to the mixture at room temperature, and it was stirred for 3 hours. After that, sodium sulfite (315 mg, 2.50 mmol) and water (10 mL) were added to the reaction mixture, and it was extracted with ethyl acetate (10 mL). An organic layer was washed with water (5 mL) twice. The organic layer was concentrated under reduced pressure, and the obtained residue was subjected to purification by means of a silica gel column to obtain (benzylsulfinyl)benzene (138 mg, yield: 26%), {[chloro(phenyl)methyl]sulfinyl}benzene (337 mg, yield: 53.9%) and (benzylsulfonyl)benzene (64 mg, yield: 11%).
(Benzylsulfinyl)benzene: ¹H-NMR (300MHz, CDCl₃) δ 3.95-4.13 (2H, m), 6.95-7.01 (2H, m), 7.22-7.50 (8H, m)
{[Chloro(phenyl)methyl]sulfinyl}benzene: ¹H-NMR (300MHz, CDCl₃) δ 5.48 (1H, s), 6.89-7.50 (10H, m)
(Benzylsulfonyl)benzene: ¹H-NMR (300MHz, CDCl₃) δ 4.31 (2H, s), 7.03-7.68 (10H, m)

### (Example 8)

1-methoxy-4-(methylthio)benzene (1.00 g, 6.48 mmol) and cyanuric acid (84 mg, 0.65 mmol) were mixed with toluene (20 mL). 10% aqueous solution of sodium hypochlorite (11.1 g, 14.9 mmol) was added to the mixture at room temperature, and it was stirred for 1 hour. After that, sodium sulfite (408 mg, 3.24 mmol) and water (10 mL) were added to the reaction mixture, and it was extracted with ethyl acetate (10 mL). An organic layer was concentrated under reduced pressure, and the obtained residue was subjected to purification by means of a silica gel column to obtain 1-methoxy-4-(methylsulfonyl)benzene (1.16 g, yield: 96%).
¹H-NMR (300MHz, CDCl₃) δ 3.03 (3H, s), 3.89 (3H, s), 7.00-7.05 (2H, m), 7.84-7.90 (2H, m)

### (Example 9)

1-(methylthio)-4-nitrobenzene (1.00 g, 5.91 mmol) and cyanuric acid (76 mg, 0.59 mmol) were mixed with toluene (20 mL). 10% aqueous solution of sodium hypochlorite (10.1 g, 13.6 mmol) was added to the mixture at room temperature, and it was stirred for 2 hours. After that, sodium sulfite (372 mg, 2.96 mmol) and water (10 mL) were added to the reaction mixture, and it was extracted with ethyl acetate (10 mL). An organic layer was concentrated under reduced pressure, and the obtained residue was subjected to purification by means of a silica gel column to obtain 1-(methylsulfonyl)-4-nitrobenzene (1.13 g, yield: 95%).
¹H-NMR (300MHz, CDCl₃) δ 3.13 (3H, s), 8.15-8.20 (2H, m), 8.41-8.47 (2H, m)

### (Example 10)

1-chloro-4-(methylthio)benzene (800 mg, 5.04 mmol) and cyanuric acid (65 mg, 0.50 mmol) were mixed with toluene (16 mL). 10% aqueous solution of sodium hypochlorite (8.63 g, 11.6 mmol) was added dropwise to the mixture at room temperature, and it was stirred for 1 hour. After that, sodium sulfite (318 mg, 2.52 mmol) and water (10 mL) were added to the reaction mixture, and it was extracted with ethyl acetate (10 mL). An organic layer was concentrated under reduced pressure, and the residue was subjected to purification by means of a silica gel column to obtain 1-chloro-4-(methylsulfonyl)benzene (0.94 g, yield: 98 %).
¹H-NMR (300MHz, CDCl₃) δ 3.06 (3H, s), 7.25-7.59 (2H, m), 7.86-7.93 (2H, m)

### (Example 11)

2-(methylthio)benzothiazole (1.00 g, 5.52 mmol) and cyanuric acid (71 mg, 0.55 mmol) were mixed with toluene (20 mL). 10% aqueous solution of sodium hypochlorite (13.6 g, 18.2 mmol) was added to the mixture at room temperature, and it was stirred for 2.5 hours. After that, sodium sulfite (696 mg, 5.52 mmol) and water (10 mL) were added to the reaction mixture, and it was extracted with ethyl acetate (10 mL). An organic layer was concentrated under reduced pressure, and the residue was subjected to purification by means of a silica gel column to obtain 2-(methylsulfonyl)benzothiazole (1.17 g, yield: 99.7 %).
¹H-NMR (300MHz, CDCl₃) δ 3.42 (3H, s), 7.57-7.68 (2H, m), 7.99-8.05 (1H, m), 8.19-8.24 (1H, m)

### (Example 12)

(Allylthio)benzene (1.00 g, 6.66 mmol) and cyanuric acid (86 mg, 0.67 mmol) were mixed with toluene (20 mL). 10% aqueous solution of sodium hypochlorite (13.9 g, 18.6 mmol) was added dropwise to the mixture at room temperature, and it was stirred for 2 hours. After that, water (10 mL) was added to the reaction mixture, and it was extracted with ethyl acetate (10 mL). An organic layer was concentrated under reduced pressure, and the residue was subjected to purification by means of a silica gel column to obtain (allylsulfonyl)benzene (0.58 g, yield: 48 %).
¹H-NMR (300MHz, CDCl₃) δ 3.81 (2H, d, J 7.4 Hz), 5.10-5.19 (1H, m), 5.30-5.36 (1H, m), 5.71-5.85 (1H, m), 7.50-7.69 (3H, m), 7.84-7.90 (2H, m)

### (Example 13)

Tetrahydrothiophene (1.00 g, 11.3 mmol) and cyanuric acid (146 mg, 1.13 mmol) were mixed with toluene (20 mL). 10% aqueous solution of sodium hypochlorite (19.3 g, 26.0 mmol) was added to the mixture at room temperature, and it was stirred for 1 hour. After that, sodium sulfite (712 mg, 5.65 mmol) was added to the reaction mixture, and it was extracted with ethyl acetate (10 mL). An aqueous layer was extracted with ethyl acetate (10 mL) 5 times, and a combined organic layer was concentrated under reduced pressure. The residue was subjected to purification by means of a silica gel column to obtain tetrahydrothiophene 1,1-dioxide (1.31 g, yield: 96%).
¹H-NMR (300MHz, CDCl₃) δ 2.13-2.30 (4H, m), 2.95-3.07 (4H, m)

### (Example 14)

2-(methylthio)pyridine (1.00 g, 7.99 mmol) and cyanuric acid (103 mg, 0.80 mmol) were mixed with toluene (20 mL). 10% aqueous solution of sodium hypochlorite (13.7 g, 18.4 mmol) was added to the mixture at room temperature, and it was stirred for 3 hours. After that, the mixture was subjected to separation, and an organic layer was concentrated under reduced pressure. The residue was subjected to purification by means of a silica gel column to obtain 2-(methylsulfonyl)pyridine (614 mg, yield: 49 %) and 2-[(trichloromethyl)sulfonyl]pyridine (234 mg, yield: 11%).
2-(methylsulfonyl)pyridine: ¹H-NMR (300MHz, CDCl₃) 6 3.24 (3H, s), 7.53-7.60 (1H, m), 7.94-8.03 (1H, m), 8.10 (1H, d, J 7.9 Hz), 8.72-8.76 (1H, m)
2-[(trichloromethyl)sulfonyl]pyridine: ¹H-NMR (300MHz, CDCl₃) δ 7.25-7.74 (1H, m), 8.02-8.09 (1 H, m), 8.3 (1H, d, J 7.9 Hz), 8.80-8.94 (1H, m)

### (Example 15)

2-chloro-6-methylthiopyridine (1.00 g, 6.26 mmol) and succinimide (62 mg, 0.63 mmol) were mixed with toluene (20 mL). 10% aqueous solution of sodium hypochlorite (11.6 g, 15.7 mmol) was added dropwise to the mixture at 0 to 5°C, and it was stirred for 1 hour. After that, ethyl acetate (10 mL), water (10 mL) and sodium sulfite (473 mg, 3.76 mmol) were added to the mixture, and it was stirred. After stirring and separation, an organic layer was concentrated under reduced pressure, and the residue was subjected to purification by means of a silica gel column to obtain 2-chloro-6-methylsulfonylpyridine (1.18 g, yield: 98%).
¹H-NMR (300MHz, CDCl₃) δ 3.26 (3H, s), 7.57-7.60 (1H, m), 7.90-8.05 (2H, m)

### (Example 16)

2-chloro-6-methylthiopyridine (208 mg, 1.30 mmol) and N-chlorosuccinimide (17 mg, 0.13 mmol) were mixed with ethyl acetate (2 mL). 10% aqueous solution of sodium hypochlorite (2.2 g, 2.93 mmol) was added to the mixture at room temperature, and it was stirred for 1 hour. After that, ethyl acetate (10 mL) and sodium sulfite (164 mg, 1.30 mmol) were added to the mixture, and it was stirred. After stirring and separation, an organic layer was concentrated under reduced pressure, and the residue was subjected to purification by means of a silica gel column to obtain 2-chloro-6-methylsulfonylpyridine (244 mg, yield: 98 %).

### (Example 17)

2-cyano-6-methylthiopyridine (1.00 g, 6.66 mmol) and succinimide (66 mg, 0.67 mmol) were added to a mixed solution of ethyl acetate (10 mL) and water (1 mL). 10% aqueous solution of sodium hypochlorite (11.2 g, 15.0 mmol) was added dropwise to the mixture at 20 to 30°C, and it was stirred for 1 hour. Sodium sulfite (839 mg, 6.66 mmol) was added to the reaction mixture, and it was stirred. After stirring and separation, an aqueous layer was extracted with ethyl acetate (10 mL). An organic layer was combined therewith and concentrated under reduced pressure. The residue was subjected to purification by means of a silica gel column to obtain 2-cyano-6-methylsulfonylpyridine (1.14 g, yield: 94 %).
¹H-NMR (300MHz, CDCl₃) δ 3.32 (3H, s), 7.92-7.95 (1H, m), 8.15-8.21 (1H, m), 8.29-8.35 (1H, m)

### (Example 18)

2-cyano-6-methylthiopyridine (1.00 g, 6.66 mmol) and N-chlorosuccinimide (89 mg, 0.67 mmol) were mixed with ethyl acetate (10 mL). 10% aqueous solution of sodium hypochlorite (11.2 g, 15.0 mmol) was added to the mixture at 0 to 10°C, and it was stirred for 2 hours. After that, ethyl acetate (10 mL) and sodium sulfite (839 mg, 6.66 mmol) were added to the mixture, and it was stirred for 1 hour. After stirring and separation, an organic layer was concentrated under reduced pressure, and the residue was subjected to purification by means of a silica gel column to obtain 2-cyano-6-methylsulfonylpyridine (1.14 g, yield: 94%).

### (Comparative Examples)

Regarding production of sulfone from sulfide, the method in Example 1 was compared with conventional oxidation methods using halogens (Comparative Examples 1 to 3).

### (Comparative Example 1)

Diphenylsulfide (500 mg, 2.68 mmol) was dissolved in toluene (10 mL). 10% aqueous solution of sodium hypochlorite (5.99 g, 8.05 mmol) was added to the mixture at room temperature, and it was stirred for 18 hours. After that, sodium sulfite (1.01 g, 8.05 mmol) and water (10 mL) were added to the reaction mixture, and it was extracted with ethyl acetate (10 mL). An organic layer was subjected to quantitative determination using HPLC, and it was confirmed that diphenylsulfide (394 mg, 79 %) remained and diphenylsulfoxide (110 mg, 20 %) and diphenylsulfone (6 mg, 1 %) were produced.

### (Comparative Example 2)

Diphenylsulfide (500 mg, 2.68 mmol) and methyltrioctylammoniumchloride (542 mg, 1.34 mmol) were dissolved in toluene (10 mL). 10% aqueous solution of sodium hypochlorite (5.99 g, 8.05 mmol) was added to the mixture at room temperature, and it was stirred for 18 hours. After that, sodium sulfite (1.01 g, 8.05 mmol) and water (10 mL) were added to the reaction mixture, and it was extracted with ethyl acetate (10 mL). An organic layer was subjected to quantitative determination using HPLC, and it was confirmed that diphenylsulfide (364 mg, 73 %) remained and diphenylsulfoxide (71 mg, 13 %) and diphenylsulfone (83 mg, 14 %) were produced.

### (Comparative Example 3)

Diphenylsulfide (500 mg, 2.68 mmol) and water (6 mL) were mixed with toluene (10 mL). Trichloroisocyanuric acid (624 mg, 2.68 mmol) was added to the mixture at room temperature, and it was stirred for 30 minutes. After that, sodium sulfite (1.01 g, 8.05 mmol) and water (10 mL) were added to the reaction mixture, and it was extracted with ethyl acetate (20 mL). An organic layer was subjected to quantitative determination using HPLC, and it was confirmed that diphenylsulfone (495 mg, 99 %) was produced.

In the reaction in which only sodium hypochlorite was used (Comparative Example 1), the reaction was very slow (18 hours), and the yield of sulfone was very low (1 %). Similarly, in the reaction in which sodium hypochlorite was used in the presence of methyltrioctylammoniumchloride, which is a phase transfer catalyst (Comparative Example 2), the reaction was very slow (18 hours), and the yield of sulfone was low (14 %). In contrast, in the case of the method in Example 1, the reaction was completed in a shorter time (2 hours) and a higher yield of sulfone (94 %) was obtained compared to Comparative Examples 1 and 2.

On the other hand, in the reaction in which only trichloroisocyanuric acid was used (Comparative Example 3), the reaction was faster (30 minutes) and a higher yield of sulfone (99 %) was obtained compared to Example 1. However, in Comparative Example 3, as a by-product, cyanuric acid was generated from trichloroisocyanuric acid used in the molar quantity equal to that of the substrate. In contrast, the amount of cyanuric acid after the reaction in Example 1 is significantly smaller than the amount in Comparative Example 3 since the catalytic amount is the same as that before the reaction (0.1 mol per 1 mol of substrate). Thus, it was demonstrated that isolation and purification of the reaction product is more easily carried out in Example 1 compared to Comparative Example 3.

Further, Examples 3 and 4 using dimethoxyethane as a solvent were compared with the following comparative example (no catalyst).

### (Comparative Example 4)

Diphenylsulfide (500 mg, 2.68 mmol) was dissolved in dimethoxyethane (10 mL). 10% aqueous solution of sodium hypochlorite (5.99 g, 8.05 mmol) was added to the mixture at room temperature, and it was stirred for 4 hours. After that, sodium sulfite (1.01 g, 8.05 mmol) and water (10 mL) were added to the reaction mixture, and it was extracted with ethyl acetate (10 mL). An organic layer was subjected to quantitative determination using HPLC, and it was confirmed that diphenylsulfide (388 mg, 78 %) remained and diphenylsulfoxide (107 mg, 20 %) and diphenylsulfone (11 mg, 2 %) were produced.

Thus, the yields of sulfoxide and sulfone when using only sodium hypochlorite were much lower compared to those in Examples 3 and 4 (71 % and 27 %, respectively).

### INDUSTRIAL APPLICABILITY

The oxidation method of the present invention is superior to conventional oxidation methods in terms of reaction time, operability and safety of reaction, yield of reaction product, easiness of isolation/purification, economic efficiency, etc., and therefore has advantages as an industrial production method of various organic oxides.

## Claims

1. A method for producing an organic oxide, wherein a substrate is oxidized using hypohalous acid, hypohalous acid salt, chlorine, bromine or iodine in the presence of water and a catalytic amount of a compound represented by the following formula (I):
R¹-X¹-NY-R²,
wherein:
X¹ represents -CO- or -SO₂-; Y represents a hydrogen atom, a potassium atom, a sodium atom, a chlorine atom, a bromine atom or an iodine atom; R¹ represents a substituted or unsubstituted hydrocarbon group, -NYR³ group or -OR³ group (in the formulae, R³ represents a substituted or unsubstituted hydrocarbon group, and Y represents the same meaning as defined above); and R² represents a hydrogen atom or -CO-R⁴ group (in the formula, R⁴ represents a substituted or unsubstituted hydrocarbon group, -NYR⁵ group or -OR⁵ group (in the formulae, R⁵ represents a substituted or unsubstituted hydrocarbon group, and Y represents the same meaning as defined above)); or R¹ and R⁴ may bind to each other to form a further substituted or unsubstituted nitrogen-containing heterocyclic ring.

2. The method according to claim 1, wherein in the compound represented by formula (I), X¹ is -CO- and R² is -CO-R⁴ group (in the formula, R⁴ represents the same meaning as defined in claim 1).

3. The method according to claim 1, wherein the compound represented by formula (I) is a compound represented by the following formula (Ia): 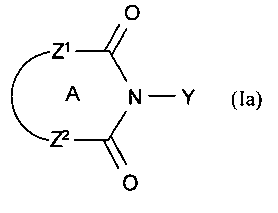 wherein: Ring A represents a further substituted or unsubstituted nitrogen-containing heterocyclic ring (in Ring A, Z¹ and Z² each independently represent a carbon atom, -NY- or -O-); and Y represents the same meaning as defined in claim 1.

4. The method according to claim 3, wherein the compound represented by formula (Ia) is a compound represented by one of the following formulae: 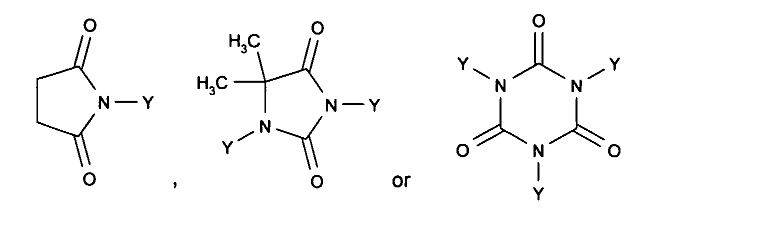 wherein Y represents the same meaning as defined in claim 3.

5. The method according to claim 1, using a hypohalous acid salt selected from the group consisting of potassium hypochlorite, calcium hypochlorite, lithium hypochlorite, sodium hypobromite, calcium hypobromite, lithium hypobromite and sodium hypoiodite.

6. The method according to claim 1, wherein the substrate is sulfide or sulfoxide.
